# EUROPÄISCHE PATENTSCHRIFT

(11) **EP 3 442 629 B1**
(45) Veröffentlichungstag und Bekanntmachung des Hinweises auf die Patenterteilung: **24.03.2021**
(21) Anmeldenummer: 17719813.2
(22) Anmeldetag: 05.04.2017
(51) Int. Cl.: A61M 15/00, G06M 1/08, G06M 1/24

(54) **HANDGERÄT ZUR AUSGABE EINER PHARMAZEUTISCHEN SUBSTANZ SOWIE ZÄHLWERK FÜR EIN SOLCHES HANDGERÄT**
HANDHELD DEVICE FOR DISPENSING A PHARMACEUTICAL SUBSTANCE, AND COUNTER FOR SUCH A HANDHELD DEVICE
APPAREIL À MAIN DESTINÉ À DISTRIBUER UNE SUBSTANCE PHARMACEUTIQUE AINSI QUE COMPTEUR POUR UN TEL APPAREIL À MAIN

(30) Priorität: 13.04.2016 DE 102016106875
(43) Veröffentlichungstag der Anmeldung: 20.02.2019
(73) Patentinhaber: Von Schuckmann, Alfred, 47627 Kevelaer (DE)
(72) Erfinder: Von Schuckmann, Alfred, 47627 Kevelaer (DE)
(74) Vertreter: Müller, Enno
(86) Internationale Anmeldenummer: PCT/EP2017/058063
(87) Internationale Veröffentlichungsnummer: WO 2017/178294

(56) Entgegenhaltungen:
- WO-A1-92/09324
- WO-A1-2009/037085
- WO-A1-2015/006292
- WO-A1-2016/091652
- US-A1- 2010 065 649
- US-A1- 2010 229 855
- US-B1- 6 679 251

## Beschreibung

### Gebiet der Technik

Die Erfindung betrifft zunächst ein Handgerät zur Ausgabe einer pharmazeutischen Substanz, mit einem Gehäuse, in dem ein Vorratsbehältnis mit der pharmazeutischen Substanz und ein Zählwerk angeordnet sind, wobei das Vorratsbehältnis durch Druckbetätigung durch einen Benutzer nach Durchlaufen eines bestimmten Verlagerungsweges die pharmazeutische Substanz ausgibt und das Zählwerk in Abhängigkeit des Durchlaufens des Verlagerungsweges eine Einwirkung zur Drehung eines Zählrades des Zählwerks erfährt, wobei das Zählwerk weiter ein zur Drehung des Zählrades ausgebildetes Antriebsteil und eine Rückstellfeder aufweist und das Zählrad drehbar an einem Halterungsteil angebracht ist, wobei darüber hinaus das Halterungsteil unabhängig von dem Gehäuse ausgebildet ist, das Antriebsteil unmittelbar an dem Halterungsteil angebracht ist und bei der Druckbetätigung zunächst die Einwirkung auf das Zählrad des Zählwerks erfolgt und hiernach die Ausgabe der pharmazeutischen Substanz.

Weiter betrifft die Erfindung ein Zählwerk für ein Handgerät zur Ausgabe einer pharmazeutischen Substanz mit einem mindestens ein lesbares Zeichen aufweisenden Zählrad und einem zur Drehung des Zählrades ausgebildeten Antriebsteil, wobei das Zählwerk eine Rückstellfeder zur Rückverlagerung nach einer Betätigung aufweist und die gesondert von dem Antriebsteil ausgebildete Rückstellfeder ein Eingriffsteil zur Zusammenwirkung mit dem Antriebsteil aufweist.

### Stand der Technik

Derartige Zählwerke sind bereits in verschiedener Hinsicht bekannt geworden. Insbesondere ist auf die WO 2007/104694 A1 (US 8,132,565 B2) zu verweisen.

Hieraus ist sowohl ein Handgerät zur Ausgabe einer pharmazeutischen Substanz als auch ein Zählwerk bekannt, welches Zählwerk im konkreten Benutzungsfall oberseitig eines Vorratsraumes des Handgerätes, welcher Vorratsraum die pharmazeutische Substanz aufnimmt, angeordnet ist. Es wird zufolge einer unmittelbaren Druckbetätigung durch den Benutzer beaufschlagt. Das Antriebsteil ist mit einer Drehachse in zwei Lagerungsausformungen aufgenommen, die innenseitig einer Betätigungstaste nach unten ragend angeformt sind. Das Zählrad ist zwischen der Betätigungstaste und einer Gehäusewandung, welche beide Teile jeweils auch Teile des Gehäuses sind, aufgenommen.

Weiter ist zum Stand der Technik auch auf die WO 2006/051073 A1 (US 7,448,342 B2) zu verweisen. Das hieraus bekannte und in einem Handgerät aufnehmbare Zählwerk ist im Benutzungszustand unterhalb eines geschlossenen Behältnisses vorgesehen, welches die pharmazeutische Substanz aufnimmt und in dem Handgerät angeordnet ist. Das Behältnis wird durch das Niederdrücken desselben betätigt. Das Zählrad ist zwischen einem unteren Ringteil und einer oberen Gehäuseabdeckung aufgenommen. Das Antriebsteil ist lose eingelegt und wirkt über ein Scheibenteil, weiter auch mittels eines Planetenrades - erst - auf das Zählrad ein.

Aus der US 2010/0229855 A1 ist ein Handgerät mit einem Zählwerk bekannt, bei welchem die Rückstellfeder oberhalb des Zählwerks, zur Anlage an dem Vorratsbehältnis, verläuft. Die Feder ist durchgehend gekrümmt ausgebildet und muss zur Betätigung unter Reduzierung der Krümmung gespreizt werden.

Aus der WO92/0934 A1 sind als Rückstellelemente zwei Federarme vorgesehen, die sich im Bereich des Zählwerkes erstrecken. Sie sind gerade durchgehend ausgebildet.

Weiter ist aus der WO2009/ 037085 A1 ein Behältnis mit einem Zählwerk bekannt, bei welchem die Rückstellfeder des Zählwerks durch federnde Arme gegeben ist, die innerhalb des Zählwerkes verlaufen. Die Federarme als Rückstellfeder sind auch durchgehend gleichsinnig gekrümmt gestaltet.

Aus der US 6,679,251 B1 ist ein Handgerät mit einem Zählwerk bekannt, bei welchem die Feder auf einem gegenüber dem Boden hervorragenden Sockel abgestützt ist. Die Rückstellfedern verlaufen insgesamt durchgehend gleichsinnig gekrümmt, annähernd halbkreisartig.

Bei dem aus der WO2015/153624 A1 bekannten Handgerät mit einem Zählwerk sind Federbeine ausgebildet, die sich am Boden abstützen. Diese Federbeine sind durchgehend gleichsinnig gekrümmt gebildet.

Aus der US 2010/065649 A1 ist ein Behältnis mit einem Zählwerk bekannt, bei welchem das Zählwerk oberhalb des Vorratsbehältnisses angeordnet ist. Die Rückstellfeder ist in einer Seitenansicht geradlinig durchgehend ausgebildet.

### Zusammenfassung der Erfindung

Ausgehend von dem dargelegten Stand der Technik beschäftigt sich die Erfindung mit der Aufgabe, ein Behältnis mit einem Zählwerk und ein Zählwerk anzugeben, die vorteilhaft ausgestaltet sind.

Diese Aufgabe ist im Hinblick auf das Behältnis beim Gegenstand des Anspruches 1 gelöst, wobei darauf abgestellt ist, dass die Rückstellfeder hebelartig ausgebildet ist und bei Betätigung um eine Schwenkachse verschwenkt, wobei in einem Querschnitt, in welchem ein größter Abstand zwischen dem Eingriffsteil und der Schwenkachse gegeben ist, die Rückstellfeder einen, gesehen vom Zählwerk, konkaven Verlauf aufweist und dass die Rückstellfeder mit Bezug auf den Querschnitt in Richtung einer Längsachse unterhalb des Zählwerks verläuft, zwischen dem Zählwerk und einem Boden des Gehäuses, mit einem zur wippenartigen Auslegung der Rückstellfeder sich auf dem Boden des Gehäuses abstützenden Federbereich, so dass bei einer Betätigung des Handgerätes das federarmendseitige Eingriffsteil im Wesentlichen entgegen der Verlagerungsrichtung des Vorratsbehältnisses und des Zählwerkes angehoben wird, hierbei dem Antriebsteil entgegenkommend.

Die Rückstellfeder kann weiter einen im Querschnitt annähernd geradlinig bis hin zu kreislinienabschnittsförmig nach innen, hin zu einem mit dem Halterungsteil eingeschlossenen spitzen Winkel, gekrümmt verlaufenden Federabschnitt aufweisen.

Es kann sichergestellt werden, dass eine Abgabe von Substanz nicht ohne Zählung erfolgt. Es kann sich gegebenenfalls eine Situation einstellen, bei welcher sich eine Zählung ergibt, ohne dass Substanz oder eine wesentliche Menge an Substanz abgegeben wurde. Eine Irritation des Nutzer dahingehend, dass dieser mehr Substanz eingenommen hat, als tatsächlich gezählt wurde, lässt sich aber praktisch ausschließen.

Hinsichtlich des Querschnitts ist bezogen auf eine Nutzungsstellung des Zählwerks in einem Handgerät auf einen Querschnitt entlang einer Längsachse, also einem Längsschnitt, abgestellt, insbesondere auf einen Längsschnitt, in dem sich die genannte Rückstellfeder mit einer größten Länge abbildet. Der größte Abstand zwischen dem Eingriffsteil und der Schwenkachse der Rückstellfeder kann sich auf ein Abstandsmaß in Erstreckungsrichtung der Längsachse betrachtet beziehen.

Es ist ein Handgerät angegeben, bei welchem im Zuge einer Druckbetätigung des Vorratsbehälters zur Ausgabe einer pharmazeutischen Substanz zunächst im Verlauf der hierbei einhergehenden Linearverlagerung des Vorratsbehälters relativ zu dem Gehäuse eine Zählung, d.h. Weiterführung des Zählrades des Zählwerkes, erfolgt, bevor die pharmazeutische Substanz aus dem Vorratsbehälter und dem Handgerät heraus ausgegeben wird. Die Ausgabe der pharmazeutischen Substanz kann mit einer anschlagbegrenzten Endstellung bezüglich der Verlagerbarkeit des Vorratsbehälters im Gehäuse einhergehen. Im Falle einer solchen anschlagbegrenzten Endstellung erfolgt die Zählung, d.h. das Weitersetzen des Zählrades um eine Einheit vor Erreichen dieser Anschlagbegrenzung.

Die gegenüber der Substanzausgabe voreilende Beaufschlagung des Zählrades ist bevorzugt erreicht zufolge entsprechender Ausgestaltung des Zählwerkes, insbesondere der dem Zählwerk zugeordneten Rückstellfeder. Diese verläuft ausgehend von einem Anbindungsbereich der Rückstellfeder bspw. an dem Halterungsteil, welche Anbindung zugleich die Schwenkachse der Rückstellfeder bilden kann, im Wesentlichen konkav (betrachtet vom Zählwerk aus in Richtung Rückstellfeder). Der konkave Verlauf kann durch eine kreisbogenförmige Gestaltung mit über die Länge gleichbleibendem oder sich änderndem Radius gegeben sein. Auch kann der konkave Verlauf sich durch eine Aneinanderreihung von Kreislinienabschnitten unterschiedlicher Radien und/oder geradlinig gestreckter Abschnitt zusammensetzen. Einzelne Kreislinienabschnitte können für sich gesehen durchaus auch konvex verlaufend gebildet sein. Wesentlich ist in diesem Zusammenhang der insgesamt konkave Verlauf der Rückstellfeder.

Der konkave Verlauf lässt den zur Verfügung stehenden Raum günstig ausnutzen. Es ergibt sich auch ein vergleichsweise großer, auf einem Boden aufliegender oder sich nahe des Bodens erstreckender Bereich der Feder. Zugeordnet einem freien Ende der Feder kann es im Zuge einer Bewegung des Zählwerks, die zu einem Niederdrücken bzw. Anspannen der Feder führt, auch zu einer gewissen voreilenden Annäherung an das Zählwerk kommen. Dies kann auch für sich vorteilhaft sein, wie weiter unten noch erläutert.

In einer möglichen Ausgestaltung ist der konkave Verlauf im Wesentlichen gegeben durch zwei in dem beschriebenen Querschnitt zumindest annähernd langgestreckt linienförmige Federarmabschnitte, die in einem Verbindungsbereich untereinander einen stumpfen Winkel von mehr als 90° bis hin zu 160° einschließen. Insgesamt ist eine, bevorzugt sowohl im entspannten als auch gespannten Zustand, in Längserstreckung hebelartige Rückstellfeder gegeben.

Das Eingriffsteil kann, wie weiter auch bevorzugt, endseitig der Rückstellfeder im Bereich des der Schwenkachse abgewandten freien Endes angeordnet sein, weiter bevorzugt einstückig und materialeinheitlich mit der Rückstellfeder ausgebildet sein.

Mit Bezug auf den Querschnitt verläuft die Rückstellfeder in Richtung einer Längsachse unterhalb des Zählwerkes, weiter bevorzugt zwischen dem Zählwerk und einem Boden des Gehäuses.

Das Eingriffsteil erstreckt sich mit Bezug zu dem Querschnitt bevorzugt in einem Bereich der Druckfeder, der außerhalb des tiefsten Bereiches der Druckfeder ausgebildet ist. Der tiefste Bereich der Druckfeder kann, wie auch bevorzugt, der den vorbeschriebenen stumpfen Winkel einschließende Verbindungsbereich der Federarmabschnitte sein, über welchen tiefsten Federabschnitt eine Abstützung der Rückstellfeder auf einem Gehäuseabschnitt, bspw. Gehäuseboden, erreicht werden kann.

In der Nutzungsstellung ist so eine wippenartige Hebelwirkung im Bereich der Rückstellfeder erreichbar. Ein Absenken des Zählwerkes im Zuge der entsprechenden Verlagerung des Vorratsbehältnisses in dem Gehäuse führt zufolge entsprechender Beaufschlagung der Druckfeder und damit einhergehender Verschwenkung derselben um deren Schwenkachse bei Abstützung auf dem Gehäuseboden zu einem entgegen der Verlagerungsrichtung von Vorratsbehälter und Zählwerk gerichteten Anhebung des Eingriffsteils. Somit wird in bevorzugter Ausgestaltung nicht nur das Antriebsteil bei einer Absenkverlagerung des Vorratsbehälters und des Zählwerkes in Richtung auf das Eingriffsteil verlagert, sondern vielmehr überlagert hierzu auch das Eingriffsteil in Richtung auf das Antriebsteil angehoben. Hierdurch kann eine voreilende Zählung erreicht werden, bevor die Gesamtverlagerungsstrecke des Vorratsbehälters zur Ausgabe der pharmazeutischen Substanz vollendet ist.

Weitere Merkmale der Erfindung sind nachstehend, auch in der Figurenbeschreibung und der Zeichnung, oftmals in ihrer bevorzugten Zuordnung zu den bereits erläuterten Konzepten beschrieben bzw. dargestellt. Sie können aber auch in einer Zuordnung zu nur einem oder mehreren einzelnen Merkmalen, die hier beschrieben oder zeichnerisch dargestellt sind, oder unabhängig oder in einem anderen Gesamtkonzept von Bedeutung sein.

Insbesondere kann jedes der vorstehend herausgestellten Merkmale auch in einem der weiteren Konzepte jeweils verwirklicht sein, bis hin zu einer Zusammenfassung aller vorstehend beschriebenen Merkmale bei einem Gegenstand.

Der zur wippenartigen Auslegung der Rückstellfeder sich bspw. auf einem Gehäuseboden abstützende Federbereich kann der beschriebene Verbindungsbereich zweier sich bspw. im Wesentlichen etwa linear erstreckender Federarmabschnitte sein. Auch kann dieser Abstützbereich gegeben sein durch einen Teilbereich eines kreislinienabschnittförmigen Federabschnittes, wobei weiter im Zuge der Federbeaufschlagung bei Verlagerung des Vorratsbehälters und des Zählwerkes der konkrete Abstützpunkt auf dem Gehäuseboden o. dgl. entlang dem konkaven Verlauf des Federarmes, insbesondere bei einem kreislinienabschnittförmigen Verlauf, wandern kann.

Jedenfalls geht die lineare Verlagerung eines unmittelbar mit der Schwenkachse an dem Zählwerk angebundenen Rückstellfederabschnittes mit einer Verlagerung des das Eingriffsteil tragenden Rückstellfederabschnittes in im Wesentlichen entgegengesetzter Richtung einher.

Der zur ordnungsgemäßen Funktion des Zählwerkes nötige Verlagerungsweg des Eingriffsteiles relativ zu dem Antriebsteil, der im bekannten Stand der Technik dem Gesamtverlagerungsweg des Vorratsbehälters in dem Gehäuse entspricht, addiert sich zufolge der vorliegenden Lösung aus einem Teil des Gesamtverlagerungsweges des Vorratsbehälters mit dem Zählwerk und des Eingriffsteilverlagerungsweges, der aus einer überlagerten Bewegung des Eingriffsteiles entgegenkommend zu dem Antriebsteil resultiert.

Die Rückstellfeder ist bevorzugt auch im entspannten Zustand in Anlage an dem Gehäuse, insbesondere auf einem Boden des Gehäuses des Handgerätes.

Insbesondere ist auch bevorzugt, das Sperrteil einteilig, weiter bevorzugt materialeinheitlich einteilig bereits mit dem Halterungsteil auszugestalten. Somit hat das Halterungsteil noch eine weitere Funktion übernommen, was weiter dazu beiträgt, ein Zählwerk mit möglichst wenigen Einzelteilen zur Verfügung zu stellen.

Das Zählrad kann, wie schon angesprochen, zylindrisch ausgebildet sein, mit einer bevorzugt an einer im Benutzungszustand unteren Randkante ausgebildeten Eingriffsverzahnung. Dadurch, dass die Eingriffsverzahnung bevorzugt an der unteren Randkante des weiter bevorzugt zylindrischen Zählrades ausgebildet ist, kann die verbleibende (Außen-)Fläche (nach oben) zur Aufbringung der lesbaren Zeichen frei genutzt werden.

Die zylindrische Ausbildung des Zählrades, ohne dass hierin, im Inneren des so gebildeten Zylinderkörpers, auch weitere Einbauteile vorgesehen sind, ist auch günstig zur Zusammenwirkung mit einem Vorratsbehältnis des Handgerätes, in welchem die pharmazeutische Substanz aufgenommen ist. Im Inneren des Zählrades befinden sich bevorzugt nur Abschnitte des Halterungsteils. Das Vorratsbehältnis ist typischerweise als sogenannter Kanister ausgebildet, der eine im Längsquerschnitt untere stufenförmige Gestaltung, mit einem mittigen zapfenartigen Vorsprung aufweist, aus welchem Vorsprung dann letztlich ein rohrförmiges Ventilteil herausragt, aus dem die Substanz bei einer Betätigung ausgebracht wird.

Die zylindrische Ausgestaltung des Zählrades kann insbesondere so vorgesehen sein, dass eine obere Stirnfläche des Zylinderkörpers der unteren äußeren Stufenfläche des genannten Vorratsbehältnisses zugeordnet angeordnet sein kann.

Das Vorratsbehältnis ist typischerweise gegen eine in dem Vorratsbehältnis selbst angebrachte Feder zur Betätigung von einem Benutzer nach unten zu drücken, womit dann zufolge der Druckausübung auf das Zählrad beziehungsweise das das Zählrad aufnehmende Halterungsteil die Bewegung erfolgt, welche letztlich zufolge des wippenartig über die Feder entgegen des verlagerten Eingriffsteils zur voreilenden Betätigung des Zählwerkes vor dem Ausstoß der pharmazeutischen Substanz führt.

Das Halterungsteil ist bevorzugt in gleicher Weise zylindrisch ausgebildet.

Auch kann das Halterungsteil das Zählrad in einem Längsquerschnitt von innen übergreifen. Somit liegt das Zählrad außenseitig mit seiner Außenfläche frei. Diese Außenfläche trägt bevorzugt die schon angesprochenen lösbaren Zeichen.

Das Antriebsteil kann bevorzugt in weiterer Einzelheit als Schneckenwelle mit Verzahnungsvorsprüngen ausgebildet sein. Dies bedeutet, dass einerseits eine Umfangsverzahnung vorgesehen ist, diese Umfangsverzahnung an dem Antriebsteil jedoch an einem zu einer Drehachse des Antriebsteils schräg verlaufenden, sich schneckenartig um die Drehachse erstreckenden Wendelgang ausgebildet ist.

Das Antriebsteil ist bevorzugt derart angeordnet, dass seine Drehachse quer zu einer Mittelachse des Zählrades und/oder des Halterungsteiles, die bevorzugt eine Zylinderachse ist, bei entsprechender Ausbildung des Zählrades und/oder des Halterungsteils, verläuft. Darüber hinaus kann die Drehachse, wie auch bevorzugt, parallel zu der geometrischen Schwenkachse der Rückstellfeder ausgerichtet sein.

Das Eingriffsteil wirkt bevorzugt auf der dem Zählrad abgewandten Seite der Drehachse des Antriebsteiles auf das Antriebsteil ein. Die beschriebenen Verzahnungsvorsprünge dienen auch gerade der Zusammenwirkung zwischen dem Eingriffsteil und dem Antriebsteil sowie auch dem Sperrteil.

Das Sperrteil wirkt bevorzugt bezüglich des Zählrades innenseitig des Halterungsteiles bzw. auf einer dem Zählrad zugewandten Seite der Drehachse des Antriebsteils auf dieses ein. Somit ist zunächst eine gegenüberliegende Einwirkung - bezogen auf die Drehachse des Sperrteils und des Antriebsteils - erreicht. Zugleich können die genannten Teile gegeneinander fahren, ohne dass die Gefahr einer Behinderung gegeben ist.

Das Sperrteil und das Eingriffsteil sind bevorzugt gegensinnig zueinander gerichtet angeordnet. Die freien Enden des Sperrteils und des Eingriffsteils sind zueinander gerichtet angeordnet. Hierbei kann im Einzelnen - bezogen auf einen beschriebenen Einbauzustand, bei welchem das Zählwerk von oben von dem Vorratsbehältnis beaufschlagt ist - das Sperrteil oberseitig und das Eingriffsteil unterseitig angeordnet sein.

Das Eingriffsteil und/oder das Sperrteil können - bezogen auf eine Ansicht von oben oder unten, in welcher sich eine Mittelachse des Zählrades und/oder des Halterungsteils punktförmig abbildet - außerhalb zu dem Zählrad beziehungsweise dem Halterungsteil im Übrigen angeordnet sein. Die Anordnung außerhalb des Halterungsteils im Übrigen bezieht sich darauf, dass die Anbindung für das Eingriffsteil, die bevorzugt durch die Rückstellfeder selbst gegeben ist, beziehungsweise die Anbindung für das Sperrteil, die insoweit dann auch Teile des Halterungsteils sind, bereits, soweit sie in der genannten Ansicht sichtbar sind, als außerhalb des Halterungsteils liegend angesehen werden können.

Die Rückstellfeder kann auch noch in der genannten Ansicht über das Eingriffsteil und/oder das Sperrteil seitlich hinausreichen.

Die Rückstellfeder selbst ist bevorzugt in Form eines Bügels, insbesondere eines geschlossenen Bügels, gebildet. Der Bügel weist zwei Enden auf, die an dem Halterungsteil befestigt sind. Die Befestigungsbereiche, beispielsweise in Form von Anbindungspunkten, können sich hinsichtlich einer Durchmesserlinie des bevorzugt zylindrisch gebildeten Halterungsteils, etwa bezogen auf den durch die Halterungswände gegebenen Zylinder, gegenüberliegen. Sie können in Bezug eines hierdurch gebildeten Kreises des Zylinders auch auf einer Sekante sich gegenüberliegen. In letzterem Fall sind die Befestigungsbereiche beziehungsweise die Anbindungspunkte bevorzugt derart versetzt zu der Mittelachse angeordnet, dass die Mittelachse zwischen der Sekante und dem Sperrteil und/oder dem Eingriffsteil liegt, dieses wiederum bezogen auf die Ansicht, beispielsweise von oben, in welcher sich die Mittelachse punktförmig abbildet. Die Anbindungspunkte bzw. die unmittelbar diese Anbindungspunkte umgebenden Bereiche des federseitigen Bügels können die geometrische Schwenkachse bilden.

Das Zählrad ist bevorzugt als einteiliges Zylinderteil gebildet.

### Kurze Beschreibung der Zeichnungen

Nachstehend ist die Erfindung weiter anhand der beigefügten Zeichnung erläutert, die aber lediglich zwei Ausführungsbeispiele darstellt. Hierbei zeigt:
- Fig. 1: eine perspektivische Außenansicht eines Handgerätes mit darin angeordnetem Vorratsbehältnis und Zählwerk;
- Fig. 2: einen Querschnitt durch den Gegenstand der Figur 1, geschnitten entlang der Ebene II-II, eine unbelastete Grundstellung von Vorratsbehältnis und Zählwerk betreffend;
- Fig. 3: eine Herausvergrößerung des Bereichs III-III in Figur 2, bei in Ansicht dargestelltem Zählwerk;
- Fig. 4: eine der Figur 3 entsprechende Darstellung, eine Zwischenstellung im Zuge der Betätigung des Handgerätes betreffend;
- Fig. 5: eine Folgedarstellung zu Figur 4, die Betätigungsendstellung betreffend;
- Fig. 6: das Zählwerk in perspektivischer Einzeldarstellung;
- Fig. 7: das Zählwerk in einer perspektivischen Explosionsdarstellung; und

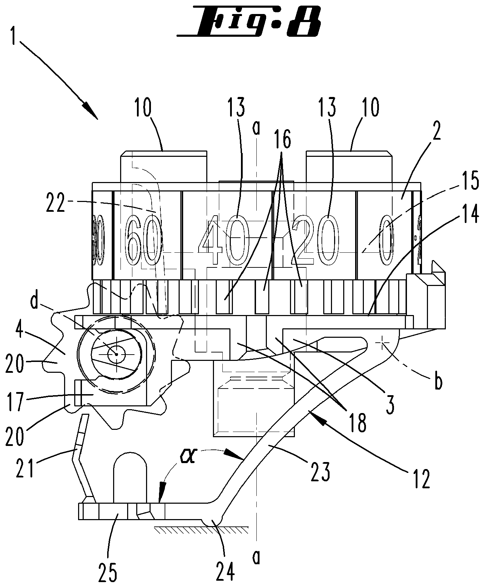

- Fig. 8: das Zählwerk in Ansicht;
- Fig. 9: eine der Figur 3 entsprechende Darstellung, betreffend eine zweite Ausführungsform.

### Beschreibung der Ausführungsformen

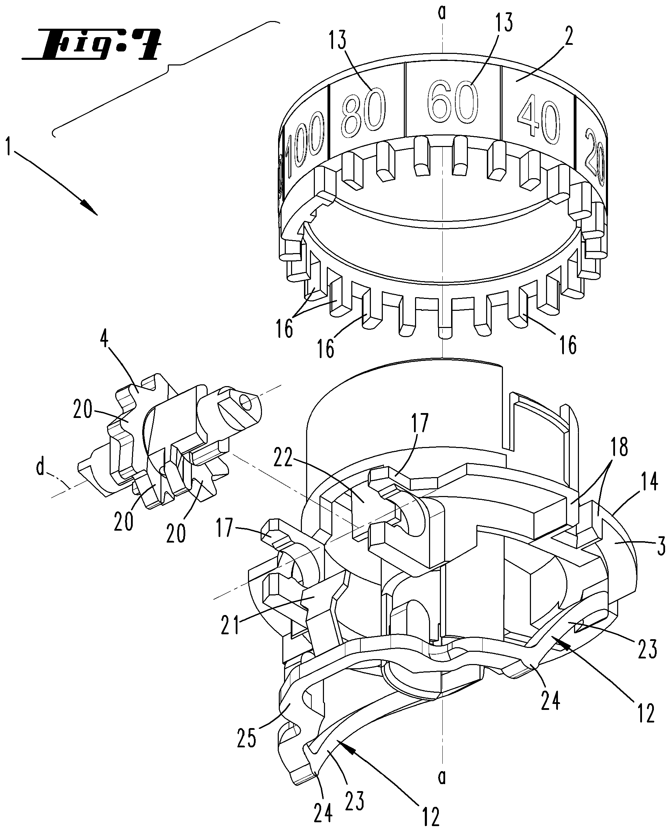

Dargestellt und beschrieben ist, vergleiche insbesondere Figur 7, ein Zählwerk 1, das aus einem Zählrad 2, einem Halterungsteil 3 und einem Antriebsteil 4 besteht.

Schon diese wenigen Teile, ersichtlich bevorzugt nur drei Teile, sind wesentlich für dieses Zählrad. Insbesondere ist von Bedeutung, dass das Halterungsteil 3 mit zusätzlichen Funktionen und Funktionsteilen zugleich, bevorzugt einteilig materialeinheitlich, ausgebildet ist.

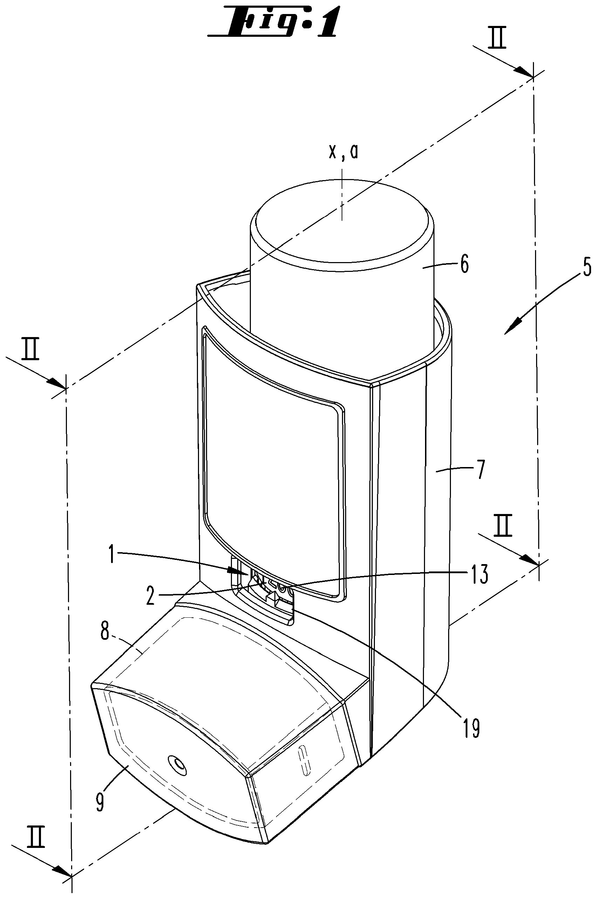

Das Zählwerk 1 ist in einem Handgerät 5, wie es grundsätzlich in Figur 1 wiedergegeben ist, angeordnet.

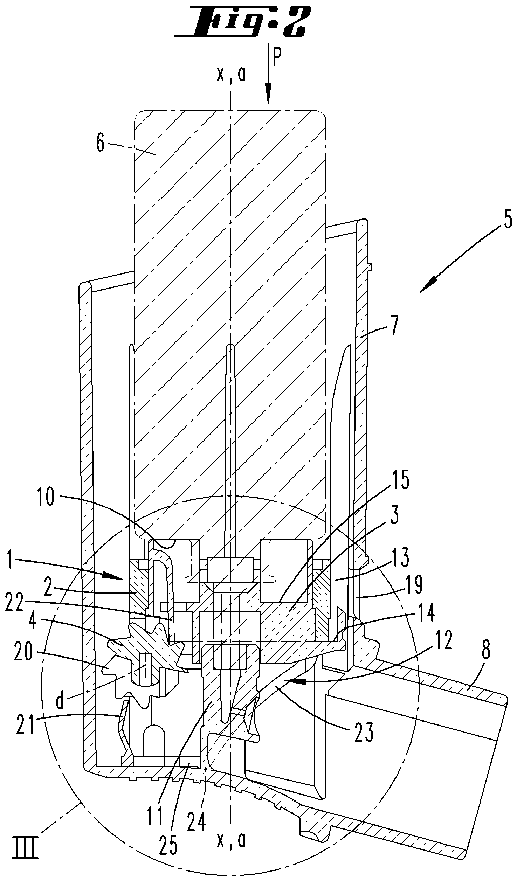

Wie sich weiter aus der Querschnittsdarstellung der Figur 2 ergibt, kann in dem Handgerät 5 ein Vorratsbehältnis 6 angeordnet sein, in dem auszugebende pharmazeutische Substanz enthalten ist. Insbesondere handelt es sich bei dem Handgerät 5 um eine Inhaliervorrichtung.

Das Handgerät 5 weist weiter ein Gehäuse 7 auf, mit einem im Benutzungszustand vertikalen Teil, in welchem das Vorratsbehältnis 6 aufgenommen ist und einem hiervon winklig, bspw. recht- oder stumpfwinklig, bezogen auf eine Längsachse x des vertikalen Teils und einer Seitenansicht, abragenden Mundstück 8. Das Mundstück 8 kann im nicht benutzten Zustand von einer Verschlusskappe 9 überfangen sein.

Das Zählwerk 1 ist insgesamt in dem Gehäuse 7 des Handgerätes 5 aufgenommen.

Das Zählwerk 1 kann, wie in Figur 2 auch angedeutet, insbesondere unterseitig des im Längsquerschnitt stufenartig unterseitig gebildeten Vorratsbehältnis 6 angeordnet sein, und zwar derart, dass es mit dem Halterungsteil 3 unmittelbar in Anlage kommen kann zu einer Stufenfläche, und zwar bevorzugt einer äußeren Stufenfläche 10 des Vorratsbehältnisses 6. Das Vorratsbehältnis 6 wird zur Betätigung gegen eine in diesem selbst ausgebildete, hier im Einzelnen nicht dargestellte Feder niedergedrückt, und zwar relativ zu einem feststehenden Aufnahme- und Ausgabestumpf 11 in dem Gehäuse 7.

Hierbei wird entsprechend das Halterungsteil 3 zusammen mit dem Zählrad 2 gegen eine bevorzugt an dem Halterungsteil 3 auch ausgebildete Rückstellfeder 12 niedergedrückt. Bei diesem Niederdrücken wirkt das Antriebsteil 4 mit dem Zählrad 1 zusammen, um dieses in Bezug auf eine Mittelachse a des Zählrades 1, welche auch eine Zylinderachse sein kann, wie nachstehend noch angesprochen, drehend anzutreiben.

Das beschriebene Zählwerk 1 ist vornehmlich nicht zu einer Anzeige einer Einzelzählung bei jeder Betätigung des Handgerätes 5 vorgesehen. Es kann allerdings auch ein Einzelzählwerk sein. Bevorzugt zeigt das Zählwerk 1 nur nach einer bestimmten Anzahl von Betätigungen eine neue Ziffer (vollständig) an, beispielsweise nach 5,10 oder 20 jeweiligen Betätigungen.

Das Zählrad 2 weist entsprechende lesbare Zeichen 13, wie dargestellt auf einer Umfangsaußenfläche der bevorzugt zylindrischen Gestaltung des Zählrades 2, auf.

Die Zeichen 13 sind durch ein Fenster 19 im Gehäuse 7 lesbar.

Das Zählrad 2 ist an dem Halterungsteil 3 gehaltert. Hierzu kann das Halterungsteil 3 das Zählrad 2 mit Halterungsfingern und/ oder einer Halterungswand hintergreifen. Die Halterungsfinger können elastisch ausbiegbar vorgesehen sein. Unterseitig kann das Zählrad 2 auf einer Stufenfläche 14 des Halterungsteils 3 aufsitzen. Das Halterungsteil 3 weist weiter bevorzugt einen Boden 15 auf. Der Boden 15 weist darüber hinaus bevorzugt eine Halterungsöffnung auf, mit welcher das Zählwerk 1 im Einbauzustand, vgl. Fig. 2, an dem Aufnahme-/ Ausgabestumpf 11 des Gehäuses 7 rastgehaltert sein kann.

Weiter bevorzugt ist der Boden 15 etwa auf der Höhe der Stufenfläche 14 ausgebildet.

In der Halterungsöffnung können noch Rastfinger ausgebildet sein, welche eine mögliche Halterung des Zählwerks an dem Aufnahme-/ Ausgabestumpf 11 des Gehäuses der Vorrichtung begünstigen können.

Der Aufnahmestumpf 11 kann hierzu eine umlaufende Nut aufweisen, in der solche Rastfinger eingreifen können. Im Zuge einer Betätigung kann sich der Boden 15 und das Zählwerk 1 insgesamt relativ zu dem Gehäuse 7 und insbesondere relativ zu dem Aufnahme-/ Ausgabestumpf 11 um die vertikale Höhe (Breite) der Nut bewegen.

Das Zählrad 2 ist unterseitig mit einer Eingriffsverzahnung 16 ausgebildet. Hierüber kann zur Drehung auf das Zählwerk 1 eingewirkt werden.

Die Einwirkung auf die Eingriffsverzahnung 16 erfolgt durch das Antriebsteil 4.

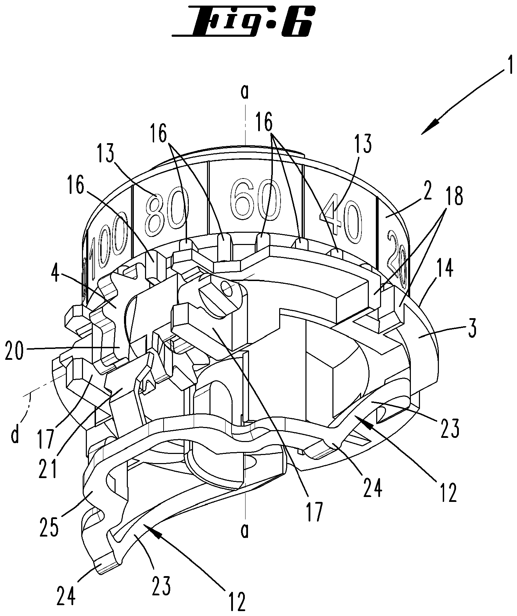

Das Halterungsteil 3 ist bevorzugt auch kreisringförmig, weiter bevorzugt zylindrisch, gegebenenfalls in einer Höhenrichtung einer Zylinderachse, die bevorzugt mit der Mittelachse a zusammenfällt, mit unterschiedlichen Bereichen ausgebildet. Unterhalb der Stufenfläche 14 ist vorzugsweise ein Funktionsabschnitt ausgebildet. In diesem Funktionsabschnitt, der bevorzugt auch ringförmig und weiter bevorzugt zylinderförmig ist, sind bevorzugt eine oder mehrere Ausformungen vorgesehen, die eine oder mehrere Funktionen erbringen können. Zunächst eine Halterungsausformung 17 zur Halterung des Antriebsteils 4. Es kann sich um Rastöffnungen für eine Achse des Antriebsteils 4 handeln. Weiter rippenartige Gegenausformungen 18 zur Halterung in dem Gehäuse 7 des Handgerätes 5. Darüber hinaus die Anbindung der Rückstellfeder 12. Diese kann von einer unteren Stirnfläche des Funktionsabschnittes ausgehend sich erstrecken. Sie schließt in ihrer Erstreckungsrichtung mit der Mittelachse a, bezogen auf eine Seitenansicht gemäß Figur 2, in der sich die Mittelachse a als Linie und die Drehachse d des Antriebsteils 4 als Punkt darstellt, bevorzugt zunächst einen spitzen Winkel zu dem Funktionsabschnitt ein. Die Rückstellfeder 12 ist bevorzugt bügelartig gebildet (siehe Figuren 6 und 7), weiter bevorzugt derart, dass der Bügel in der genannten Seitenansicht mit seinen beiden Bügelarmen in Überdeckung ist, so dass im Querschnitt bzw. in einer entsprechenden Ansicht nur der Bügelarm sichtbar ist.

Die Rückstellfeder 12 ist weiter bevorzugt hinsichtlich der bügelartigen Ausbildung geschlossen ausgebildet. Zwei Endbereiche des Bügels sind bevorzugt an unterschiedlichen Stellen unterseitig des Halterungsteils 3 im Übrigen (also für diese Zwecke gesehen ohne die Rückstellfeder 12) in dieses übergehend gebildet.

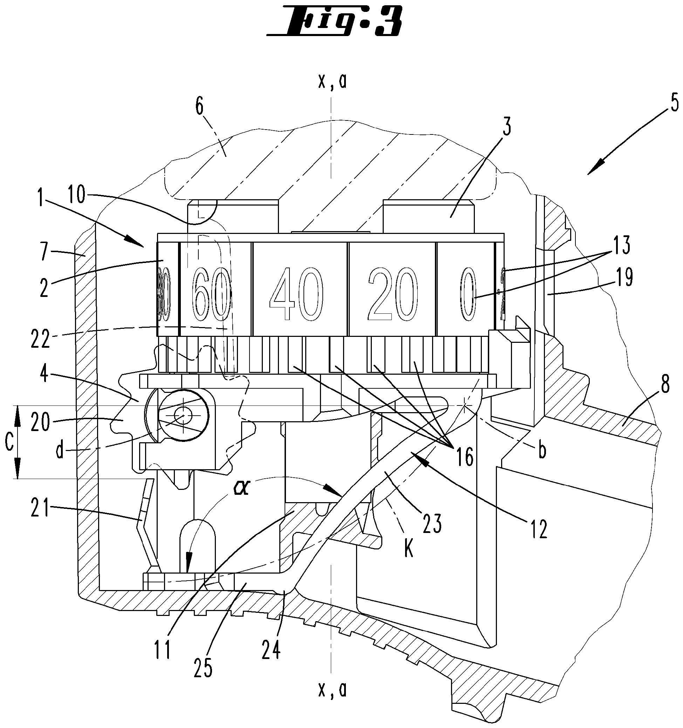

Insgesamt ergibt sich gesehen vom Zählwerk 1 bevorzugt ein konkaver Verlauf K der Rückstellfeder 12 bzw. dessen in Überdeckung liegender Bügelarme (vgl. Fig. 3). So kann sich zunächst ausgehend vom Anbindungsbereich an dem Funktionsabschnitt, wobei sich in diesem Anbindungsbereich eine geometrische Schwenkachse b bildet, ein im Querschnitt annähernd geradlinig bis hin zu kreislinienabschnittförmig nach innen hin zum eingeschlossenen spitzen Winkel zum Funktionsabschnitt gekrümmt verlaufender Federarmabschnitt 23 ergeben. Dieser Federarmabschnitt 23 kann innerhalb des insgesamt konkaven Verlaufs der Rückstellfeder 12 geradlinig bis hin zu konvex verlaufen.

Der Federarmabschnitt 23 erstreckt sich bis zu einem Abstützabschnitt 24, über welchen sich die Rückstellfeder 12 insgesamt auf dem Boden des Gehäuses 7 abstützt. Mit Bezug auf den Querschnitt gemäß Figur 2 überschreitet hierbei der Federarmabschnitt 23 - ausgehend von dem Anbindungsbereich im Bereich der Schwenkachse b - die Mittelachse a. Auch kann der Abstützabschnitt 24 unmittelbar im Bereich der Mittelachse a oder auch vor dieser auf dem Gehäuseboden aufsitzen.

In Verlängerung des Federabschnittes 23 über den Abstützabschnitt 24 hinaus kann sich ein im Querschnitt geradlinig verlaufender zweiter Federabschnitt 25, der im Bereich des Abstützabschnittes 24 zu dem ersten Federabschnitt 23 einen Winkel α von etwa 120 bis 140° einschließen kann, ergeben.

In einer unbelasteten Grundstellung gemäß der Darstellung in den Figuren 2 und 3 kann eine Abstützung des Federabschnittes 25 über dessen annähernd gesamte Länge auf dem Gehäuseboden gegeben sein. Auch kann die Abstützung allein über den Abstützabschnitt 24 gegeben sein.

Im Bereich des dem Abstützabschnitt 24 abgewandten freien Endes des von dem Abstützabschnitt 24 frei abragenden Federabschnittes 25 ist ein zur Einwirkung auf das Antriebsteil 4 ausgebildetes Eingriffsteil 21 befestigt.

Das Antriebsteil 4 ist in weiterer Einzelheit als Schneckenwelle, bevorzugt eingängige Schneckenwelle, ausgebildet. Eine Schneckenwendelsteigung kann angepasst an die Beabstandung zweier benachbarter Zahnvorsprünge des Zählrades 2 gebildet sein. Hierdurch, und durch die bezüglich einer Umdrehung vorgegebene Mitnahme um einen bestimmten Betrag eines Zahnvorsprunges, kann die tatsächliche Zählwirkung in Bezug auf eine jeweilige Betätigung eingestellt werden.

Ein jeweiliger Mitnahmevorsprung 20, der zugleich auch als Zahn zur Einwirkung des Eingriffsteils 21 dienen kann, kann im Einzelnen zwischen zwei Mitnahmevorsprüngen 20 treten und aufgrund der wendelförmigen beziehungsweise schneckenartigen Gestaltung dann bei einer Drehung eine Mitnahme des Zählrades 2 bewirken.

Der Eingriffsteil 21 ist bevorzugt an der Rückstellfeder 12 ausgebildet, und zwar weiter bevorzugt materialeinheitlich mit der Rückstellfeder 12. Im Hinblick auf den konkaven Verlauf K der Rückstellfeder 12 insgesamt wird das Eingriffsteil 21 nicht als Teil der Rückstellfeder betrachtet.

Wie darüber hinaus beispielsweise aus Figur 6 ersichtlich, kann das Eingriffsteil 21 an einem quer zu der Drehachse d des Antriebsteils 4 am weitesten vorspringenden und am weitesten von einer Schwenkachse b entfernten Bereich der Rückstellfeder 12 angebracht sein, demgegenüber aber bevorzugt weiter vorkragend entgegen der Richtung auf die Mittelachse a.

Das Eingriffsteil 21 ist in einem Querschnitt quer zur Mittelachse a bevorzugt schwertartig, gerade verlaufend, gebildet. Auch kann oberseitig, am freien Ende des Eingriffsteils 21 eine gewisse gekrümmte oder abgeknickte Ausformung vorgesehen sein (vgl. Figur 3).

In der in Figur 3 dargestellten unbelasteten Grundstellung ergibt sich ein in Richtung der Längsachse x betrachteter größter Abstand c zwischen dem freien Ende des Eingriffsteils 21 und der Schwenkachse b der Rückstellfeder 12.

Bei einer Betätigung des Handgerätes 5, also beim Ausführungsbeispiel einem Niederdrücken des Vorratsbehältnisses 6 in Richtung der in den Figuren dargestellten Druckbetätigung P, wird über die wippenartige Ausgestaltung der Rückstellfeder 12 mit ihrer Abstützung auf dem Gehäuseboden im Bereich des Abstützabschnittes 24 das federarmendseitige Eingriffsteil 21 im Wesentlichen entgegen der Verlagerungsrichtung des Vorratsbehältnisses 6 und des Zählwerkes 1 angehoben, hierbei dem Antriebsteil 4 entgegenkommend.

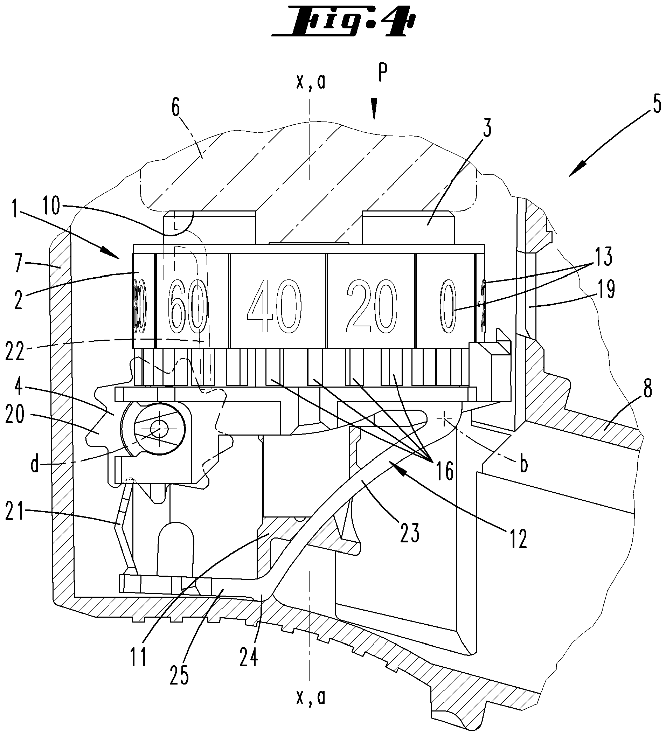

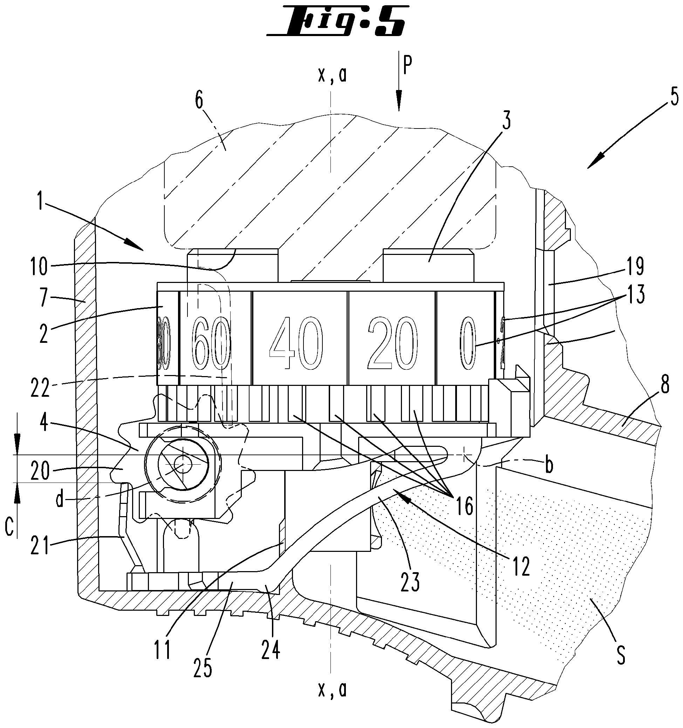

Bei einer Betätigung des Handgerätes 5 trifft der Eingriffsvorsprung 21 auf einen Mitnahmevorsprung 20 des Antriebsteils 4 und bewirkt eine Drehung der Schneckenwelle um die Drehachse d des Antriebsteils 4, noch bevor eine Ausgabestellung des Vorratsbehältnisses 6 erreicht wird (vgl. Figuren 4 und 5). Die Ausgabestellung bedeutet hierbei, insbesondere bei den dargestellten Ausführungsformen, dass ein Ventil in dem Vorratsbehältnis 6 durch die Druckbetätigung P in eine Offenstellung verlagert ist und so Substanz S aus dem Vorratsbehältnis 6 durch das Mundstück 8 ausströmen kann. In dieser Stellung kann sich der kleinstmögliche Abstand c zwischen dem freien Ende des Eingriffsteil 21 und der Schwenkachse b der Rückstellfeder 12 einstellen.

Zugleich wird eine Rückdrehung des Antriebsteils 4 gehindert durch ein Sperrteil 22. Auch das Sperrteil 22 wirkt mit der Schneckenwelle, konkret den die Schneckenausbildung bildenden Zahnvorsprüngen des Antriebsteils 4, zusammen.

Die Zusammenwirkung des Sperrteils 22 mit dem Antriebsteil 4 ist - bezogen etwa auf eine Seitenansicht gemäß Figur 3, in welcher sich die Drehachse d des Antriebsteils 4 punktförmig abbildet -, gegenüberliegend zu der entsprechenden Zusammenwirkung des Antriebsteils 4 mit dem Eingriffsteil 21.

Das Sperrteil 22 und das Eingriffsteil 21 sind bevorzugt entgegengesetzt gerichtet ausgebildet. Es kann sich jeweils um in Richtung der Mittelachse a und etwa parallel hierzu sich erstreckende Flachteile handeln. Das Eingriffsteil 21 und/oder das Sperrteil 22 kann oberseitig beziehungsweise zugeordnet dem jeweiligen freien Ende noch eine hakenförmige Ausbildung aufweisen, zur günstigeren Zusammenwirkung mit einem Mitnahmevorsprung 20.

Innenseitig kann das Gehäuse 7 des Handgerätes über den Umfang verteilte Rippenausformungen aufweisen. Diese Rippenausformungen dienen zur Drehsicherung des Zählwerks 1. Sie können hierzu mit den Gegenausformung 18 an dem Halterungsteil 3 zusammenwirken. Sie können weiter auch eine Führungsbegrenzung für die Rückstellfeder 12 bilden.

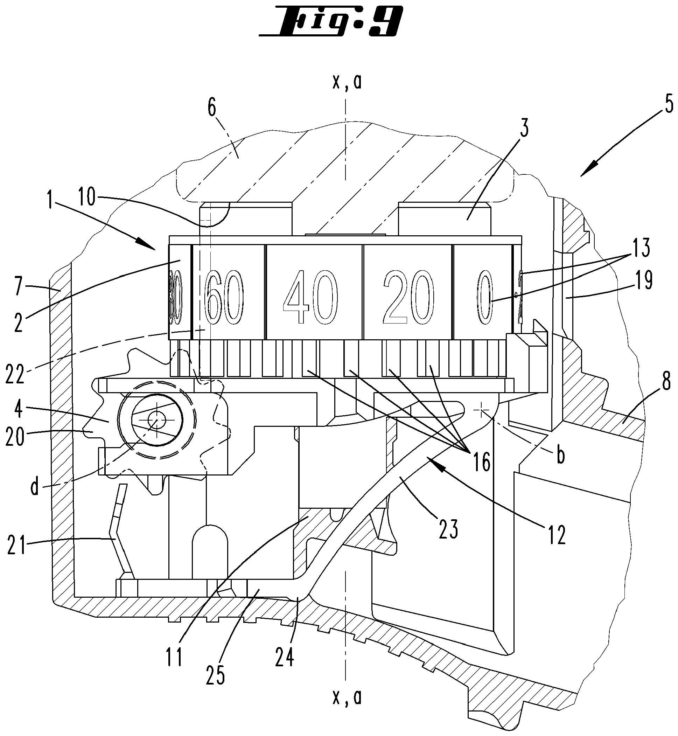

In Figur 9 ist eine alternative Ausführungsform dargestellt, bei welcher die Drehachse d des Antriebsteiles 4 außerhalb des Zählrades 2 verläuft, dies sowohl im Hinblick auf eine Projektion des Zählrades 2 in Richtung der Mittelachse a des Zählrades 2 als auch im Hinblick auf eine Projektion der Drehachse d des Antriebsteiles 4 quergerichtet zur Mittelachse a.

Wie weiter aus den Darstellungen ersichtlich, fallen die Längsachse x des Gehäuses 7 und die Mittelachse a des Zählwerks 1 in bevorzugter Ausgestaltung bei in dem Gehäuse 7 eingesetztem Zählwerk 1 zusammen.

Die vorstehenden Ausführungen dienen der Erläuterung der von der Anmeldung insgesamt erfassten Erfindungen, die den Stand der Technik zumindest durch die folgenden Merkmalskombinationen jeweils auch eigenständig weiterbilden, nämlich:

Ein Handgerät, das dadurch gekennzeichnet ist, dass bei der Druckbetätigung zunächst die Einwirkung auf das Zählrad 2 des Zählwerks 1 erfolgt und hiernach die Ausgabe der pharmazeutischen Substanz.

Ein Zählwerk, das dadurch gekennzeichnet ist, dass die Rückstellfeder 12 hebelartig ausgebildet ist und bei Betätigung um eine Schwenkachse b verschwenkt, wobei in einem Querschnitt, in welchem ein größter Abstand zwischen dem Eingriffsteil 21 und der Schwenkachse b gegeben ist, die Rückstellfeder 12 einen, gesehen vom Zählwerk 1, konkaven Verlauf aufweist.

Ein Zählwerk, das dadurch gekennzeichnet ist, dass an dem Halterungsteil 3 ein Sperrteil 22 zur Einwirkung auf das Antriebsteil (4) angebracht ist und dass das Sperrteil 22 bezüglich des Zählrades (2) innenseitig des Halterungsteils 3 ausgebildet ist.

Alle offenbarten Merkmale sind (für sich, aber auch in Kombination untereinander) erfindungswesentlich. In die Offenbarung der Anmeldung wird hiermit auch der Offenbarungsinhalt der zugehörigen/beigefügten Prioritätsunterlagen (Abschrift der Voranmeldung) vollinhaltlich mit einbezogen, auch zu dem Zweck, Merkmale dieser Unterlagen in Ansprüche vorliegender Anmeldung mit aufzunehmen. Die Unteransprüche charakterisieren mit ihren Merkmalen eigenständige erfinderische Weiterbildungen des Standes der Technik, insbesondere um auf Basis dieser Ansprüche Teilanmeldungen vorzunehmen.

**Liste der Bezugszeichen**

| | | | |
|---|---|---|---|
| 1 | Zählwerk | | |
| 2 | Zählrad | a | Mittelachse |
| 3 | Halterungsteil | b | Schwenkachse |
| 4 | Antriebsteil | c | Abstand |
| 5 | Handgerät | d | Drehachse |
| 6 | Vorratsbehältnis | x | Längsachse |
| 7 | Gehäuse | K | konkaver Verlauf |
| 8 | Mundstück | P | Druckbetätigung |
| 9 | Verschlusskappe | S | Substanz |
| 10 | Stufenfläche | | |
| 11 | Aufnahme-/ Ausgabestumpf | α | Winkel |
| 12 | Rückstellfeder | | |
| 13 | Zeichen | | |
| 14 | Stufenfläche | | |
| 15 | Boden | | |
| 16 | Eingriffsverzahnung | | |
| 17 | Halterungsausformung | | |
| 18 | Gegenausformung | | |
| 19 | Fenster | | |
| 20 | Mitnahmevorsprung | | |
| 21 | Eingriffsteil | | |
| 22 | Sperrteil | | |
| 23 | Federarmabschnitt | | |
| 24 | Abstützabschnitt | | |
| 25 | Federarmabschnitt | | |

## Patentansprüche

1. Handgerät (5) zur Ausgabe einer pharmazeutischen Substanz mit einem Gehäuse (7), in dem ein Vorratsbehältnis (6) mit der pharmazeutischen Substanz (S) und ein Zählwerk (1) angeordnet sind, wobei das Vorratsbehältnis (6) durch Druckbetätigung (P) durch einen Benutzer nach Durchlaufen eines bestimmten Verlagerungsweges die pharmazeutische Substanz ausgibt und das Zählwerk (1) in Abhängigkeit des Durchlaufens des Verlagerungsweges eine Einwirkung zur Drehung eines Zählrades (2) des Zählwerks (1) erfährt, wobei das Zählwerk (1) weiter ein zur Drehung des Zählrades (2) ausgebildetes Antriebsteil (4) und eine Rückstellfeder (12) aufweist und das Zählrad (2) drehbar an einem Halterungsteil (3) angebracht ist, wobei darüber hinaus das Halterungsteil (3) unabhängig von dem Gehäuse (7) ausgebildet ist, das Antriebsteil (4) unmittelbar an dem Halterungsteil (3) angebracht und bei der Druckbetätigung (P) zunächst die Einwirkung auf das Zählrad (2) des Zählwerks (1) erfolgt und hiernach die Ausgabe der pharmazeutischen Substanz (S), **dadurch gekennzeichnet, dass** die Rückstellfeder (12) hebelartig ausgebildet ist und bei Betätigung um eine Schwenkachse (b) verschwenkt, wobei in einem Querschnitt, in welchem ein größter Abstand (c) zwischen einem Eingriffsteil (21) und der Schwenkachse (b) gegeben ist, die Rückstellfeder (12) einen, gesehen vom Zählwerk (1), konkaven Verlauf (K) aufweist und dass die Rückstellfeder mit Bezug auf den Querschnitt in Richtung einer Längsachse unterhalb des Zählwerkes verläuft, zwischen dem Zählwerk und einem Boden des Gehäuses, mit einem zur wippenartigen Auslegung der Rückstellfeder sich auf dem Boden des Gehäuses abstützenden Federbereich, so dass bei einer Betätigung des Handgerätes (5) das federarmendseitige Eingriffsteil im Wesentlichen entgegen der Verlagerungsrichtung des Vorratsbehältnisses (6) und des Zählwerkes (1) angehoben wird, hierbei dem Antriebsteil (4) entgegenkommend.

2. Zählwerk (1) nach Anspruch 1, **dadurch gekennzeichnet, dass** ein im Querschnitt annähernd geradlinig bis hin zu kreislinienabschnittsförmig nach innen, hin zu einem mit dem Halterungsteil (3) eingeschlossenen spitzen Winkel, gekrümmt verlaufender Federabschnitt (23) gegeben ist.

3. Zählwerk nach einem der Ansprüche 1 oder 2, **dadurch gekennzeichnet, dass** an dem Halterungsteil (3) ein Sperrteil (22) zur Einwirkung auf das Antriebsteil (4) angebracht ist und dass das Sperrteil (22) bezüglich des Zählrades (2) innenseitig des Halterungsteils (3) ausgebildet ist.

## Claims

1. Handheld device (5) for dispensing a pharmaceutical substance, with a housing (7), in which a receptacle (6) with the pharmaceutical substance (S) and a counter (1) is provided for, wherein the receptacle (6) by pressure activation (P) by a user after going through a pre-determined displacement path, dispenses the pharmaceutical substance and the counter (1) experiences, depending on the going through the displacement path, an effect for turning of a counter wheel (2) of the counter, wherein the counter (1) further has a drive part (4) for turning the counter wheel (2) and a restoring spring (12), and the counter wheel (2) is arranged turnable on a holding part (3), wherein further the holding part (3) is independent from the housing (7) and the driving part (4) is arranged immediately on the holding part (3), wherein further upon force actuation (P) first there is an effect on the counter wheel (2) of the counter (1) and thereafter the dispensing of the pharmaceutical substance (S), **characterized in that** the restoring spring (12) is lever-like and does pivot upon actuation around a pivot axis (b), wherein in a cross section in which the largest distance (c) between the engagement part (21) and the pivot axis (b) is given, the restoring spring (12) has, seen from the counter (1), a concave course (K) and that the restoring spring (12) with reference to a cross-section in direction of a longitudinal axis is provided for beneath the counter, between the counter and the bottom of the housing, with a spring area supporting on the bottom of the housing for a seesaw-like design of the restoring spring (12), so that upon actuation of the hand-held device (5) the engagement part on the side of the spring arm end is raised essentially counter to the movement direction of the receptacle (6) and the counter mechanism (1), coming nearer to the drive part.

2. Counter (1) according to claim 1, **characterized in that** there is given a spring section (23) which is in a cross section nearly straight up to a section of a circular line inwards, with an acute angle with the holding part (3).

3. Counter according to claim 1 or 2, **characterized in that** on the holding part (3) a locking part (22) is arranged for engagement to the driving part (4) and that the locking part (22) concerning the counter wheel (2) is provided for inside of the holding part (3).

## Revendications

1. Dispositif à main (5) pour la distribution d'une substance pharmaceutique, comportant un boîtier (7) dans lequel sont agencés un réservoir (6) contenant la substance pharmaceutique (S) et un dispositif de comptage (1), dans lequel le réservoir (6) distribue la substance pharmaceutique au moyen d'un actionnement par pression (P) par un utilisateur après avoir parcouru une course de déplacement déterminée et le dispositif de comptage (1) est sollicité en fonction du parcours de la course de déplacement pour faire tourner une roue de comptage (2) du dispositif de comptage (1), dans lequel le dispositif de comptage (1) comprend en outre une pièce d'entraînement (4) conçue pour faire tourner la roue de comptage (2) et un ressort de rappel (12) et la roue de comptage (2) est montée de manière rotative sur une pièce de maintien (3), dans lequel en outre la pièce de maintien (3) est conçue indépendamment du boîtier (7), la pièce d'entraînement (4) est montée directement sur la pièce de maintien (3) et, lors de l'actionnement par pression (P), la roue de comptage (2) du dispositif de comptage (1) est actionnée en premier et la substance pharmaceutique (S) est distribuée ensuite, **caractérisé en ce que** le ressort de rappel (12) est réalisé comme un levier et pivote autour d'un axe de pivotement (b) lors de l'actionnement, dans lequel dans une section transversale dans laquelle est donnée une distance maximale (c) entre une partie d'engagement (21) et l'axe de pivotement (b), le ressort de rappel (12) vu depuis le dispositif de comptage (1) a un profil concave (K), et **en ce que**, en référence à la section transversale, le ressort de rappel s'étend dans la direction d'un axe longitudinal sous le dispositif de comptage, entre le dispositif de comptage et un fond du boîtier, avec une région de ressort qui s'appuie sur le fond du boîtier pour la conception du ressort de rappel à la façon d'une bascule de manière que, lors de l'actionnement du dispositif à main (5), la partie d'engagement à l'extrémité du bras de ressort soit soulevée essentiellement dans la direction opposée à la direction de déplacement du réservoir (6) et du dispositif de comptage (1), en se rapprochant ce faisant de la pièce d'entraînement (4).

2. Dispositif de comptage (1) selon la revendication 1, **caractérisé en ce qu'**il est prévu une partie de ressort (23) approximativement rectiligne en section transversale qui s'étend de manière courbée vers l'intérieur jusqu'à former une partie de ligne circulaire et former un angle aigu avec la pièce de maintien (3).

3. Dispositif de comptage selon l'une des revendications 1 ou 2, **caractérisé en ce qu'**une partie de blocage (22) destinée à agir sur la pièce d'entraînement (4) est jointe à la pièce de maintien (3) et **en ce que** la partie de blocage (22) est formée sur le côté intérieur de la pièce de maintien (3) par rapport à la roue de comptage (2) .
